# EUROPEAN PATENT APPLICATION

(11) **EP 2 110 151 A1**
(43) Date of publication of application: **21.10.2009**
(21) Application number: 08154597.2
(22) Date of filing: 16.04.2008
(51) Int. Cl.: A61M 25/10

(54) **Double balloon occlusion device**

(71) Applicant: Diethrich, Edward, Paradise Valley, AZ 85253 (US); Frid, Noureddine, 1650 Beersel (BE)
(72) Inventor: Diethrich, Edward, Paradise Valley, AZ 85253 (US); Frid, Noureddine, 1650 Beersel (BE)
(74) Representative: Vandeberg, Marie-Paule L.G.

(57) **Abstract**

A double balloon occlusion device (22) comprising an elongated introduction device (32) having a proximal (25) and a distal (26) part. A first inflatable balloon (28) is placed coaxially with said elongated introduction device (32) towards the distal end of said elongated introduction device (32). A second inflatable balloon (34) is placed coaxially closer to the proximal end of the elongated introduction device (32) than the first balloon (28). The distance between the two balloons (28, 34) is adjustable from the proximal end of the elongated introduction device (32) on.

## Description

### Field of the invention

The invention relates to double-balloon occlusion devices intended to be used in cardiac surgery.

The invention also relates to a method for lowering the blood pressure in the heart after performing heart intervention.

### Background of the invention

A phenomenon well known by heart surgeons is the fact that at the end of an open-heart operation, they are faced with an uncontrollable rise of the systemic pressure. Though transitory, this phenomenon may be harmful to the patient, so that various methods were tested to, if not skip it, at least limit it to a duration as short as possible.

Practitioners discovered that a quick drop of the systemic blood pressure for only a brief time, at the end of an operation causes a rapid recovery to normal levels.

The most widespread technique, useful during open sternal cardiac operations, comprises a cross clamping of the superior and inferior venacavae at the junction of the right atrium. When a first clamp is applied, a drop in the systemic blood pressure is observed; the application of a second clamp produces a marked reduction. The method implies to completely block the flow of blood to the heart so that even if the left ventricle acts as a pump, there is no output. Removal of the clamps restores flow into the heart and hence cardiac output and normal systemic blood pressure. Today, there continues to be conditions where rapid control of the systemic blood pressure is desirable. For example, percutaneous aortic valve deployment demands that the pressure in the ascending aorta be reduced for accurate placement of the valve. Similarly, endografts being deployed in this region and the aortic arch can be more successfully positioned with lower blood pressure and elimination of the wind socket effect of the blood pushing the endograft distally. Even the apical (left ventricular) approach for aortic valve replacement could benefit from blood pressure control.

### Description of prior art

The two most common methods currently used for transient reduction in blood pressure are
(1) atrial pacing where very rapid pacing of the heart reduces the left ventricular output markedly and hence ensures low pressure in the ascending aorta (systemically),
(2) a pharmacologic decrease in blood pressure even temporarily placing the heart in arrest (complete standstill).

While both of these techniques are operable, there are disadvantages of each regarding cardiac irritability and lack of precise control of the blood pressure.

Ann. Thorac. Surg.January 23,2006 81:e21-e23 describes the use of rapid ventricular pacing during aortic stenting procedures. An immediate and sustained reduction in both phasic and mean blood pressure was achieved in all patients. In this procedure, two single-balloon catheters are introduced from opposite sides. The balloons are inflated in turn so as to achieve the required pressure drop.

Double-balloon catheters are known from many publications, as for example US-5 919 163, US 2005/0171472, WO 95/05209. The aim of such catheters is generally to have the possibility to manage an isolated space in a vessel so as to inject within this space various fluids. Multiple-balloons catheters are also known from e.g. US-6 685 672. The problem is that such catheters are standard-made and that neither the length separating the balloons nor the diameter of the balloons themselves can be altered. Further, when manufacturing such catheters, one is fronted with a problem of connection and feeding of the various parts.

### Summary of the invention

A first object of the invention is to provide a device allowing the simultaneous occlusion of two branches of a blood vessel, and particularly the venacava.

A further object of the invention is to provide a device as defined above that would further be very simple handle and to put in place.

A further object of the invention is that this device be adjustable to the patient's anatomy.

The subject of the invention is a double balloon occlusion device comprising an elongated introduction device having a proximal and a distal part. A first inflatable balloon is placed coaxially with said elongated introduction device towards the distal end of said elongated introduction device. A second inflatable balloon is placed coaxially closer to the proximal end of the elongated introduction device than the first balloon. The distance between the two balloons is continuously adjustable.

One of the advantages of the invention is that both balloons can be introduced simultaneously and along the same path up to their respective positions.

According to a preferred embodiment, the device is provided with a fluid injection circuit allowing the two balloons to be inflated and deflated simultaneously.

According to a preferred embodiment, the elongated introduction device comprises a first introduction catheter to which the first balloon is fastened and a second introduction catheter, extending coaxially around the first introduction catheter, to which the second balloon is fastened, both catheters being able to slide along each other, locking means able to lock the respective positions of the first and the second introduction catheters being placed towards the proximal end of the device so that the distance between the balloons can be adjusted.

An advantage of this embodiment is that it is easy to check with medical imaging devices the respective position of the balloons and to lock them in place.

According to an advantageous embodiment, the introduction catheters are comprised of an inner sub-catheter, to which the distal end of the corresponding balloon is fastened, and of an outer sub-catheter, to which the proximal end of the corresponding balloon is fastened, both sub-catheters being able to slide along each other, second and third locking means able to lock the respective positions of the inner and the outer sub-catheters being placed towards the proximal end of the device so that the length of each balloon can be adjusted, the annular space comprised between each sub-catheter being able to carry an inflating fluid provide by a fluid pressure feeding device up to the corresponding balloon.

According to a preferred embodiment, the inflation circuits of the first and second balloons are connected in parallel to the same fluid pressure feeding device.

### Short description of the drawings

These and further aspects of the invention will be explained in greater detail by way of example and with reference to the accompanying drawings wherein:
- Fig.1: is an explanatory sketch of a mechanical equivalent of the blood circuit;
- Fig.2: is a view in perspective of the catheter of the invention when put in place;

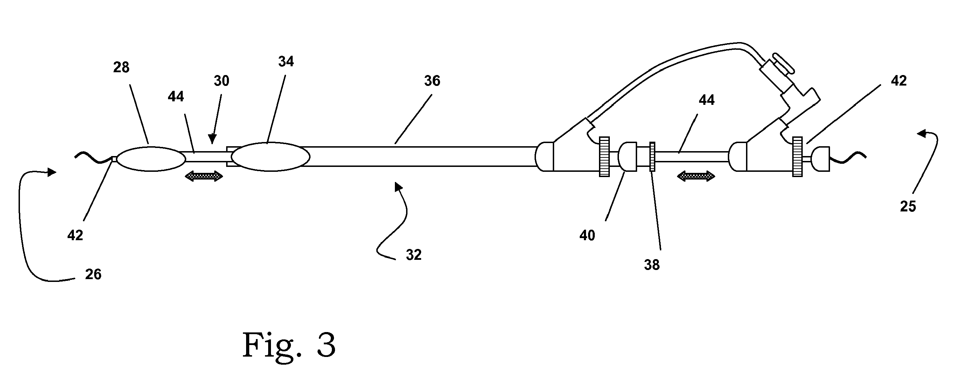

- Fig.3: is a lateral view of the catheter of the invention;
- Fig.4: is a cut view of a detail of Fig. 3;
- Fig.5: is another lateral view of the catheter of Fig. 3;
The figures are not drawn to scale. Generally, identical components are denoted by the same reference numerals in the figures.

### Detailed description of preferred embodiments

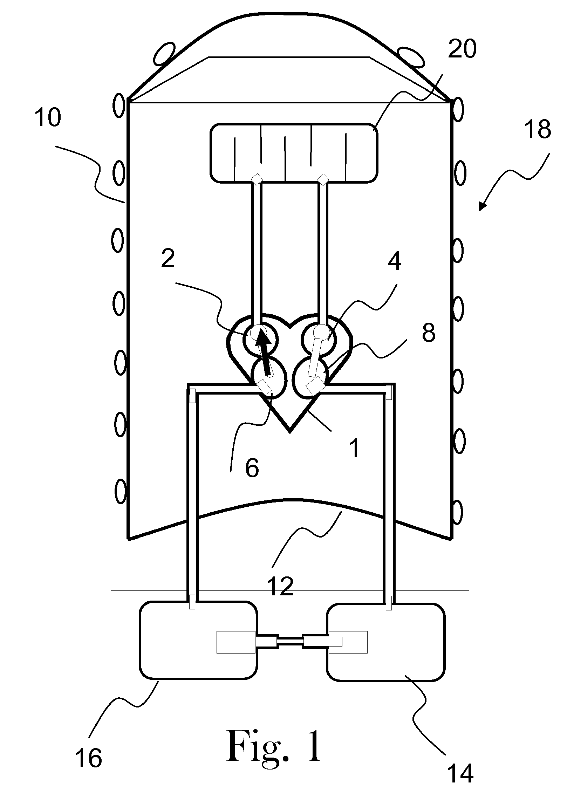

Fig.1 shows a mechanical circuit which represents schematically an equivalent of the blood circuit of a human body. This sketch is used to explain a theory on the rising of the systemic pressure. The cardiopulmonary unit, which includes the heart 1 consists schematically of two one-way chambers 2, 4 (namely the right and left atrials), two pumps 6, 8 (namely the right and left ventricles) which are surrounded by the ribcage 10 and the diaphragm 12 and lungs (which cause a changing intrathoracic pressure). To complete the diagram, the arterial and the venous network can be considered as two reservoirs 14, 16, which are located outside of the thorax 18. The flow Q of the blood is considered to be proportional to the pressure difference ΔP= P₂-P₁; the amount of blood returning to right side of the heart 2, 6 from the venous reservoir 16 is determined by the difference between intrathoracic and extrathoracic pressures. This is not the case for the left side 4, 8 of the heart 1, whose filling reservoir is the pulmonary venous reservoir 20, which is placed within the thorax 18 and therefore is not affected by the intrathoracic to extrathoracic pressure gradient.

In normal situation the intrathoracic pressure is slightly negative with respect to the atmosphere or extrathoracic pressure. It contributes to the increase of cardiac output by increasing the end-diastolic volume. (This end-diastolic volume is proportional to the pressure Pᵥ in the veins filling the ventricle).

The arterial systemic pressure Pₛₐ is based on the relation between the output flow Q and is written [Pₛₐ=RₛxQ] (Rₛ being the systemic resistance). This formula demonstrates that all these factors are linked by a fixed relationship. Hence the heart must have at its disposal other control mechanisms that allow it to regulate the blood flow.

We know that the key of success of this intrinsic control mechanism is the dependence of the cardiac output on the venous system. If we consider that the circulation system is on steady state, the right and left cardiac outputs are by definition equal. If we now assume that the [Compliance diastole x Frequency] factor is suddenly reduced, the flow Qᵣ in the right part 2, 6 of the heart 1 will be less than the flow Qₗ in the left part 4, 8 of the heart 1, so there will be a marked transfer of blood volume away from the pulmonary circulation into systemic circulation. This will raise the systemic venous pressure and lower the pulmonary venous pressure. The effect of these pressure changes will be to urge the cardiac outputs back towards balance. A marked rate of transfer of volume will persist until balance between the outputs of the two sides of the heart 1 has been restored. Then a new equilibrium will be established with a different partition of volume blood.

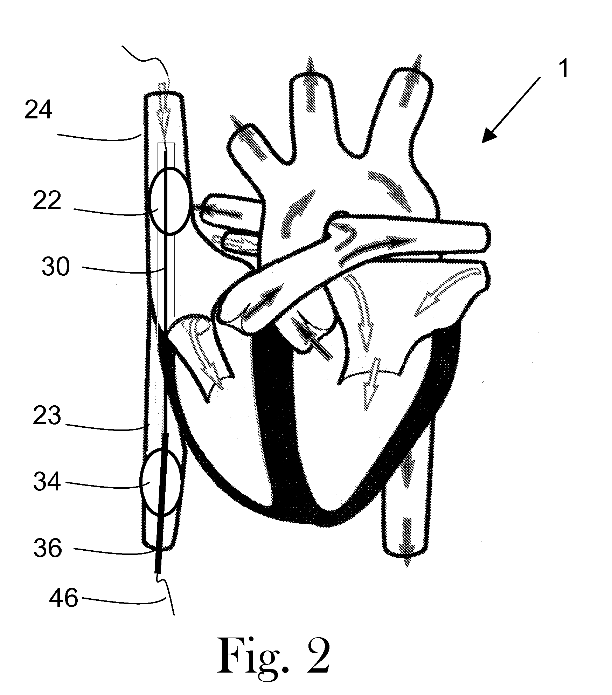

As stated above, the problem is to find an easy alternative to clamping to almost simultaneously close the inferior and superior venacavae. Already experimented solutions imply the introduction along two different paths of two distinct balloon catheters, permanently controlling their respective positions. The double-balloon catheter 22 of the invention displayed in Fig. 2 solves in an elegant way this problem, as both balloons are introduced along the same path, their relative positions in the inferior 23 and superior 24 venacavae being then adjusted in situ owing to the particular design of the catheter 22.

This catheter 22 comprises a proximal, handling part 25 and a distal part 26. A first inflatable balloon 28 is fastened coaxially towards the distal end of a first introduction catheter 30, which constitutes the first part of an elongated introduction device 32 ensuring the introduction of the balloons up to their operating place. A second inflatable balloon 34 is placed on the elongated introduction device 32, coaxially with same and closer to the proximal end of the elongated introduction device 32 than the first balloon 28. This second balloon 34 is mounted coaxially at the distal part of a second introduction catheter 36, which extending coaxially around the first introduction catheter 30. Both catheters 30, 36 are able to slide relative to each other, so that the distance between the two balloons can be adjusted at will. To ease the positioning of the balloons, non-represented marks, visible through medical imaging, are placed along the distal part of the first introduction catheter 30. The surgeon having corresponding marks placed towards the proximal part 25 of the elongated introduction device 32 can easily adjust manually the distance between the balloons and, once the best adjustment is found, lock it by acting on locking means 38 (borne here by a luer 40) so as to lock the respective longitudinal positions of the first and the second introduction catheters 30, 36.

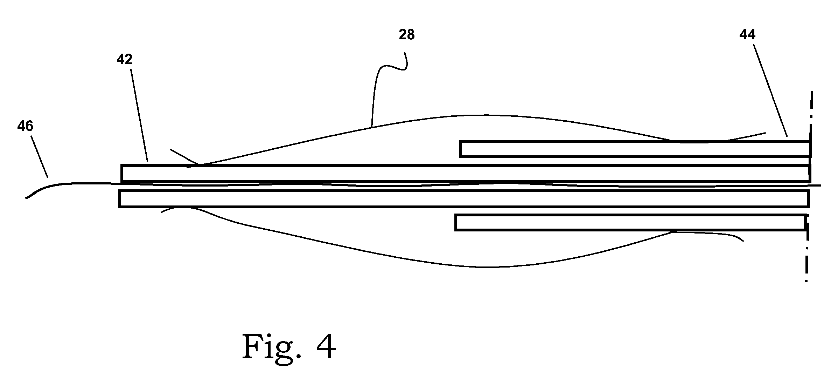

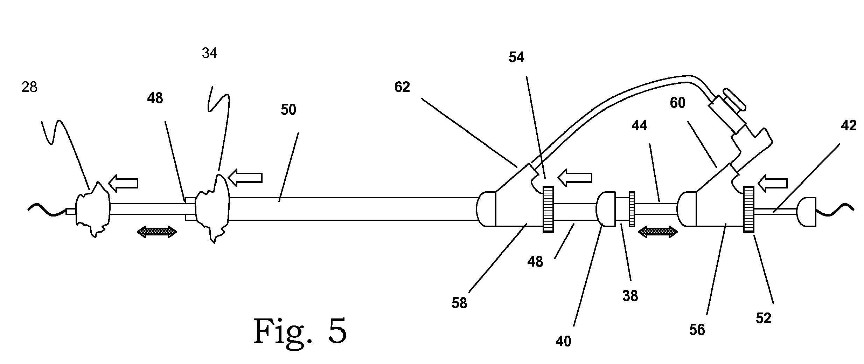

Fig. 4 is an enlarged view of a detail of a preferred embodiment of the catheter 22 of the invention. As can be seen, the first introduction catheter 30 comprises in fact two sub-catheters 42, 44 placed coaxially and slidable relative to each other. The first, inner sub-catheter 42 is hollow, so that the whole device 22 can be slid along a previously inserted guide wire 46 up to its set position. As long as it is not inflated, a balloon is roughly spindle-shaped. The distal end of the "spindle" representing the deflated first balloon 28 is fastened on this inner sub-catheter 42, while the proximal end of the balloon is fastened to the outer sub-catheter 44. A result of this original construction is that the diameter of the balloon can be adjusted. Indeed, when the operator, acting on the proximal end 25 of the device 22, slides the outer sub-catheter 44 forwards (i.e. towards the distal part of the device 22) relative to the inner sub-catheter 42 (as represented at Fig. 5), the distance between the two ends of the balloon 28 is shortened, so that, when inflated, it will adopt a larger diameter. In the embodiment shown in Fig.5, it can be seen that the same configuration can be applied to the second balloon: it suffices that the second introduction catheter also comprises two coaxial sub-catheters 48, 50, to which the respective ends of the second balloon 34 are affixed. Second series of marks borne by distal ends of the sub-catheters 42, 48 allow the operator to give the respective balloons 28, 34 their required length and hence their corresponding diameters when inflated. As provided for the distance separating the balloons, second and third locking means 52, 54 borne by luers 56, 58 placed towards the proximal end of the device 22 allow the operator to lock the respective balloons 28, 34 in a desired configuration. An advantage of the present construction is that the annular space extending between the respective pairs of sub-catheters 42, 44 and 48, 50 can carry in a very simple way the fluid (generally, gas or a physiological saline) used to inflate the balloons, avoiding the use of intricate and possibly troublesome supply pipes or conduits. To ensure the simultaneous inflation of both balloons, it suffices to connect diversion channels 60, 62 placed on the respective luers 56, 58 in parallel to a fluid supply member (not represented), that can be a simple syringe.

Another advantage of the present configuration is that when the balloons are deflated, at the end of the process, their respective diameters can be reduced by unlocking the locking means 52, 54 and sliding the inner sub-catheters backwards relative to the outer sub-catheters, thus increasing the distance between the proximal and distal ends of the balloons, that will adopt a slender spindle-shaped form.

The various operations required to control the systemic pressure at the end of a cardiac operation can thus be summarized as follows:
- bringing close to the junction of a right atrium with a superior and inferior venacavae, along a same path, a double-balloon catheter comprising a distal and a proximal end wherein the distance between a first and a second balloon is adjustable;
- adjusting the distance between the first and the second balloons so that they are placed at an adequate position in the superior and inferior venacavae respectively;
- locking the balloons at their respective set position by acting on locking means placed towards the proximal end of the catheter;
- connecting carrying conduits connected to the respective first and second balloons to fluid pressure devices;
- inflating the first and/or second balloons by injecting a fluid into carrying conduits in fluid connection with the balloons; Said operations can be completed by at least one of the following operations:

- adjusting the diameter of the respective first and second balloons by acting on their respective length along the catheter axis before inflating them.
- connecting carrying conduits connected to the respective first and second balloons to a same fluid pressure device;

It will be appreciated by persons skilled in the art that the present invention is not limited by what has been particularly shown and described hereinabove. The invention resides in each and every novel characteristic feature and each and every combination of characteristic features. Reference numerals in the claims do not limit their protective scope. Use of the verb "to comprise" and its conjugations does not exclude the presence of elements other than those stated. Use of the article "a" or "an" preceding an element does not exclude the presence of a plurality of such elements.

The present invention has been described in terms of specific embodiments, which are illustrative of the invention and not to be construed as limiting.

## Claims

1. A double balloon occlusion device (22) comprising an elongated introduction device (32) having a proximal (25) and a distal (26) part, a first inflatable balloon (28) placed coaxially with said elongated introduction device (32) towards the distal end of said elongated introduction device (32) and a second inflatable balloon (34) placed coaxially along said elongated introduction device (32), closer to the proximal end of the elongated introduction device (32) than the first balloon (28) **characterized in that** the distance between the two balloons (28, 34) is adjustable from the proximal end of the elongated introduction device (32) on.

2. A double balloon occlusion device according to claim 1, **characterized in that** each balloon (28, 34) is provided with a distinct fluid injection circuit (60, 62) allowing it to be inflated and deflated.

3. A double balloon occlusion device according to claim 2, **characterized in that** the fluid injection circuits (60, 62) of both balloons are able to be connected in parallel to the same fluid pressure feeding device, allowing the two balloons (28, 34) to be inflated and deflated simultaneously.

4. A double balloon occlusion device according to any one of claims 1 to 3, **characterized in that** the elongated introduction device (32) comprises a first introduction catheter (30) to which the first balloon (28) is fastened and a second introduction catheter(36), extending coaxially around the first introduction catheter(30), to which the second balloon (34) is fastened, both catheters (30, 36) being able to slide along each other, locking means (38) able to lock the respective positions of the first and the second introduction catheter being placed towards the proximal end (25) of the device (22) so that the distance between the balloons (28, 34) can be adjusted.

5. A double balloon occlusion device according to claim 4, **characterized in that** at least one of the introduction catheters (30, 36) is comprised of an inner sub-catheter(42, 48), to which the distal end of the corresponding balloon is fastened, and of an outer sub-catheter(44,50), to which the proximal end of the corresponding balloon is fastened, both sub-catheters (42, 44, 48, 50) being able to slide along each other, second locking means (52, 54) able to lock the respective positions of the inner and the outer sub-catheter (42, 44, 48, 50) being placed towards the proximal end (25) of the device so that the length of the corresponding balloon (28, 34) can be adjusted, the annular space comprised between each sub-catheter (42, 44, 48, 50) being able to carry an inflating fluid provide by a fluid pressure feeding device up to the corresponding balloon (28, 34).
